# EUROPEAN PATENT SPECIFICATION

(11) **EP 0 625 335 B1**
(45) Date of publication and mention of the grant of the patent: **19.11.1997**
(21) Application number: 94105973.5
(22) Date of filing: 18.04.1994
(51) Int. Cl.: A61B 17/072

(54) **Surgical stapler**
Chirurgisches Klammerinstrument
Agrafeuse chirurgicale

(30) Priority: 20.04.1993 US 49881; 14.01.1994 US 183087
(43) Date of publication of application: 23.11.1994
(73) Proprietor: United States Surgical Corporation, Norwalk, Connecticut 06856 (US)
(72) Inventor: Bolanos, Henry, East Norwalk, CT 06855 (US); Green, David T., Westport, CT 06484 (US); Ratcliff, Keith, Sandy Hook, CT 06482 (US); Sherts, Charles R., Southport, CT 06490 (US); Manzo, Scott E., Shelton, CT 06484 (US); Totakura, Nagabhushanam, Norwalk, CT 06851 (US); Pelletier, Thomas A., Wallingford, CT 06492 (US)
(74) Representative: Marsh, Roy David

(56) References cited:
- EP-A- 0 327 325
- CA-A- 2 080 326
- GB-A- 885 898
- US-A- 4 749 114
- US-A- 4 773 420
- US-A- 4 821 939
- US-A- 5 040 715

## Description

### BACKGROUND OF THE INVENTION

### Field of the Invention

The present invention relates to surgical staplers and, more particularly to surgical staplers for use in endoscopic and laparoscopic procedures.

### Discussion of the Prior Art

Laparoscopic and endoscopic surgical procedures are minimally invasive procedures in which operations are carried out within the body by means of elongated instruments inserted through small entrance openings in the body. The initial opening in the body tissue to allow passage of the endoscopic or laparoscopic instruments to the interior of the body may be a natural passageway of the body, or it can be created by a tissue piercing instrument such as a trocar. With the aid of a cannula assembly inserted into the opening, laparoscopic or endoscopic instrumentation may then be used to perform desired surgical procedures.

Laparoscopic and endoscopic surgical procedures generally require that any instrumentation inserted in the body be sealed, i.e. provisions must be made to ensure that gases do not enter or exit the body through the instrument or the entrance incision so that the surgical region of the body, e.g. the peritoneum, may be insufflated. Mechanical actuation of such instruments is for the most part constrained to the movement of the various components along a longitudinal axis with means provided to convert longitudinal movement to lateral movement where necessary. Because the endoscopic or laparoscopic tubes, instrumentation, and any required punctures or incisions are relatively narrow, endoscopic or laparoscopic surgery is less invasive and causes much less trauma to the patient as compared to procedures in which the surgeon is required to cut open large areas of body tissue.

Surgical fasteners or staples are often used to join body tissue during laparoscopic and endoscopic procedures. Such fasteners can have a pair of legs joined by a backspan and are typically set into the body by means of an elongated instrument which crimps the fastener legs to secure the fastener and tissue.

Various types of stapling instruments have been known for fixing staples to body tissue. Generally, the staples have been applied by using instruments having an anvil and an ejector mechanism for driving the legs of a staple through the tissue and against the anvil for deforming the legs into a "B" shape or the like. An example of such a stapler having an anvil is disclosed in U.S. Patent No. 4,665,916. An example of a surgical stapler having an anvil and adapted for endoscopic use is disclosed in U.S. Patent No. 5,040,715. Although these and other various types of instruments are useful for driving the legs of the staple through tissue, there are times when it is not desirable, necessary or practical to drive the legs of the staple through the body tissue in order to affix a staple. For example, when applying a purse string to tissue, as in an end to end anastomosis procedure, it is not desirable or necessary to staple through tissue, but to affix the staples to the tissue.

In cases where a purse string is to be applied to a tubular section of tissue, known stapling instruments have been rather cumbersome and complex in order to provide an anvil against which the staples can be deformed. See, for example, U.S. Patent No. 4,749,114 and 4,773,420. An anvilless surgical stapler for use in open surgery and a method of affixing a staple to tissue without completely piercing the tissue is disclosed in U.S. Patent No. 4,821,939. This document is used as basis for the preamble of claim 1. While this anvilless stapler has found success in applying purse strings in open surgery, such a stapler is not properly configured and dimensioned to be used in endoscopic or laparoscopic procedures.

Accordingly, there is a need for an endoscopic surgical stapler for applying a purse string to body tissue. Additionally, there is a need for an endoscopic surgical stapling instrument capable of applying a purse string to body tissue, wherein the staples secure the string element to body tissue without piercing the tissue with the legs of the staples.

### SUMMARY OF THE INVENTION

The present invention is defined in claim 1 below. It provides a novel anvilless endoscopic or laparoscopic surgical instrument having an endoscopic portion with proximal and distal end portions. The instrument has a pair of jaws positioned at the distal end portion, each adapted to carry a fastener cartridge. The fastener cartridge has a tissue contacting surface and a plurality of fasteners slidably disposed therein. Both the endoscopic portion and the jaws are adapted to be insertable into a cannula. Each fastener has a pair of deformable legs joined by a backspan and are disposed within and ejected from the cartridge in a manner substantially similar to that disclosed in U.S. Patent Nos. 4,821,939 and 5,158,567. Means for ejecting the fasteners may include at least one fastener firing cam member for contacting pushers disposed within the cartridge, the pushers being designed to contact and eject the fasteners from the cartridge. Means remote from the distal portion of the endoscopic instrument are adapted to cause the firing cam member to be drawn across the pusher members. This operation may be accomplished, for example, by having a slidable member disposed at the proximal end of the instrument which is attached to the cam member by means of a wire, a rod, or the like.

The purse string suture portion can be positioned adjacent the jaws and/or the tissue contacting surface of the fastener cartridge such that upon ejection of the fasteners, the suture will be held between the fastener backspans and tissue. A handle assembly having a stationary handle and a movable handle is also provided to open and close the fastener jaws.

In operation, a cannula is inserted into the abdominal cavity. The surgical instrument of the present invention is inserted through the cannula with the jaws in a substantially closed position. After insertion, the movable handle is moved away from the stationary handle thereby causing the jaws at the distal end of the instrument to open. The organ or tissue to which the purse string is to be applied is then oriented between the jaws. The movable handle is then brought into approximation with the stationary handle to cause the jaws to close about the organ or tissue. The purse string may then be applied by causing the fasteners to be ejected from the jaws, such as by causing a cam member to sequentially eject the fasteners from the cartridge, which thereby secures the suture in a purse string configuration about the organ or tissue. After firing, the jaws may then be opened to release the tissue or organ by moving the movable handle away from the stationary handle. After re-closing the jaws, the instrument may be withdrawn from the cannula. Finally, the purse string may be tightened about the organ or tissue by manipulating the suture as desired.

Preferably a stent is used to aid in the manipulation and/or approximation of tubular organs. The stent can be of a generally rectangular shape and can either be expandable or fragmentable. Preferably, corners of the stent have angular projections which facilitate placement and retention of tubular organ sections.

Furthermore, the stapler may incorporate various forms of stent embodiments to aid in the approximation and manipulation of tubular organs.

### BRIEF DESCRIPTION OF THE DRAWINGS

The foregoing objects and other features of the invention will become more readily apparent and may be understood by referring to the following detailed description of illustrative embodiments of the endoscopic or laparoscopic surgical apparatus for applying a purse string, taken in conjunction with the accompanying drawings, in which:
Figure 1 illustrates a perspective view of a preferred embodiment of the present invention.
Figure 2 illustrates an exploded view of the handle assembly of Figure 1.
Figure 3 illustrates an exploded view of the endoscopic and distal portions assembly of Figure 1.
Figure 4 illustrates a cut away plan view showing the jaws of Figure 1 in an articulated position.
Figure 5 illustrates a sectional view taken along lines 5-5 of Figure 4.
Figure 6 illustrates a side elevation cut away of the jaws of Figure 1 in an open position.
Figure 7 illustrates the jaws of Figure 6 in a closed position.
Figure 8 illustrates a perspective view of an alternative embodiment of the present invention.
Figure 9 illustrates a further alternative embodiment of the present invention.
Figure 10 illustrates the embodiment shown in Figure 9 with the handle down and jaws closed.
Figure 11 illustrates the instrument of Figure 10 after actuation of the fastener firing mechanism.
Figure 12 illustrates a cut away plan view showing jaw articulation via a push rod.
Figure 13 illustrates a sectional view taken along lines 13-13 of Figure 12.
Figure 14 illustrates a tubular section of tissue having a purse string applied thereto by the instrument of the present invention.
Figure 14a illustrates two joined tubular sections of tissue having different diameters, the smaller diameter section is cut at an angle to facilitate joining to the larger diameter section.
Fig. 15 illustrates a perspective view of an expandable stent and expandable stent applicator which may be used in combination with the instrument according to the invention;
Fig. 16 illustrates an exploded view of the stent and applicator of Fig. 15;
Fig. 17 illustrates tubular organs disposed about an unexpanded stent;
Fig. 18 illustrates tubular organs disposed about an expanded stent;
Fig. 19 illustrates a perspective view of a single shot staple applier which however forms no part of the invention;
Fig. 20 illustrates an exploded view of the single shot stapler applier of Fig. 19;
Fig. 21 illustrates a side plan cut away view of the staple applier of Fig. 19 prior to firing;
Fig. 22 illustrates a side plan cut away view of the stapler of Fig. 19 after firing;
Fig. 23 illustrates a perspective view of a surgical stapling device of the present invention, including an expandable stent applicator, where the articulation joint has not been depicted;
Fig. 24 illustrates a perspective cut away view of the surgical stapler of Fig. 23;
Fig. 25 illustrates an exploded view of a stapling assembly in accordance with one embodiment of the present invention;
Fig. 26 illustrates tubular organs being placed on a stent portion of the surgical stapler of Fig. 23;
Fig. 27 illustrates the distal end of the surgical stapler of Fig. 23 closed about two tubular organs;
Fig. 28 illustrates a cross sectional view of Fig. 27 after firing the staples;
Fig. 29 illustrates removal of the surgical stapler from the joined tubular organ.

### DETAILED DESCRIPTION OF THE PREFERRED EMBODIMENTS

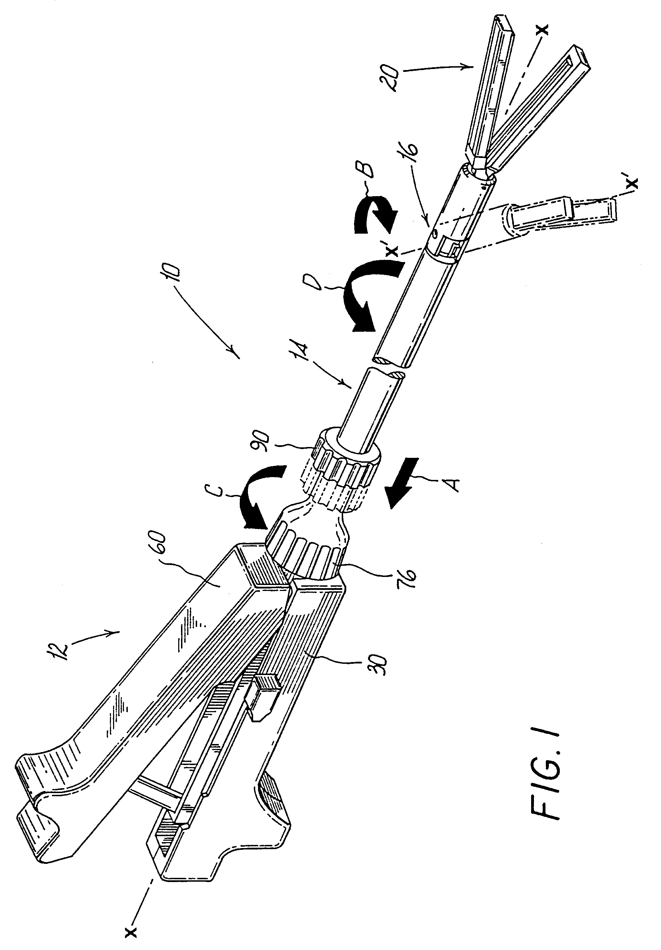

Referring now in specific detail to the drawings, in which like reference numerals identify similar or identical elements, Figure 1 illustrates a first embodiment of the endoscopic or laparoscopic surgical apparatus 10 for applying surgical fasteners. Apparatus 10 comprises endoscopic portion 14, handle assembly 12 and fastener applying assembly 20. Endoscopic portion 14 and assembly 20 are configured and dimensioned to be inserted into a cannula. Handle assembly 12 is positioned at the proximal end of endoscopic portion 14 and is shown with stationary handle 30 and movable handle 60 in an open position. Handle assembly 12 functions to open and close assembly 20, which is her detail below, endoscopic portion 14 can be articulated at articulation joint 16 by manipulating articulation knob 90. Proximal movement of knob 90 will cause the distal end of endoscopic portion 14 and assembly 20, shown in phantom, to deflect at joint 16 away from the instrument's central axis X-X. Arrows C and D depict rotational movement of the endoscopic and distal portions of instrument 10 which can be achieved by rotating knob 76.

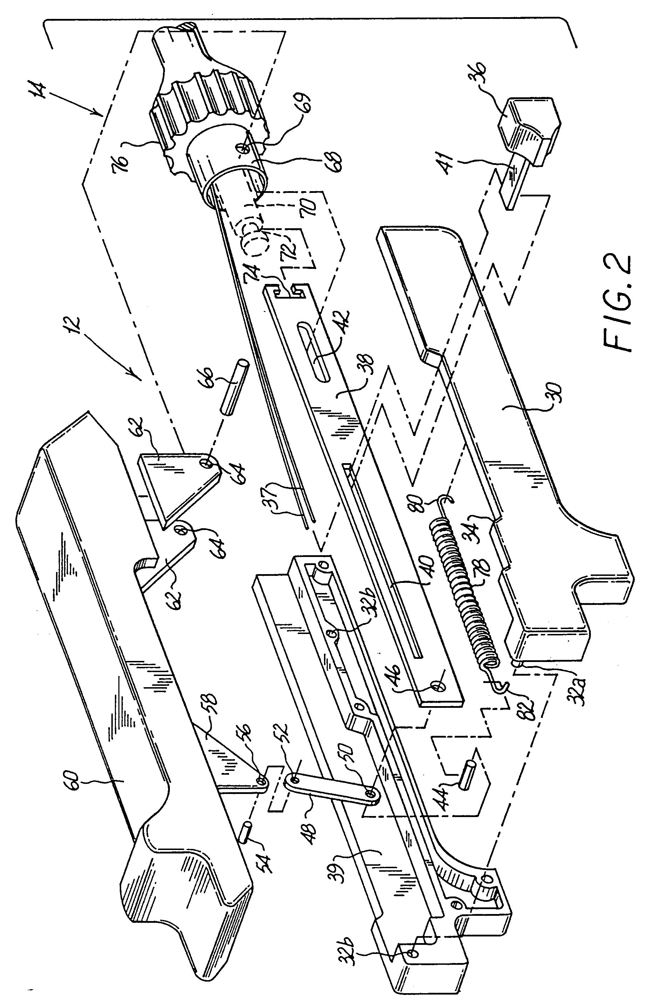

Turning to handle assembly 12 with reference to Figure 2, stationary handle housing 30 is shown in two halves. Halves 30 can be secured together by mating protrusions 32a with corresponding recesses 32b. When joined, the stationary housing defines recess 39 wherein proximal plate 38 is longitudinally movable. Plate 38 includes firing slot 40, pin opening 46, distal slot 42 and center rod opening 74. Firing slot 40 permits tongue 41 of firing button 36 to be slidably received therein. Tongue 41 is further connected to firing wires 37, the purpose of which will be described in greater detail below.

Link 48 serves to connect the proximal end of movable handle 60 with plate 38. Plate pin 44 is insertable through pin opening 46 in plate 38 and lower pin opening 50 in link 48. Movable handle pin 54 is insertable through upper pin opening 52 in link 48 and pin opening 56 in movable handle link connector plate 58. The distal end of movable handle 60 is pivotally secured to rear cover tube 68 by handle pivot pin 66. Handle pivot pin 66 passes through pivot pin openings 64 in pivot plates 62 and pin openings 69 in rear cover tube 68. The distal end of plate 38 is insertable into rear cover tube 68 and is slidable therein by means of slot 42 through which pin 66 passes. The proximal end of endoscopic portion 14 is rotatably secured to rear cover tube 68 and inner rod 70 (shown in phantom) of endoscopic portion 14 has axial groove 72 which is insertable into slot 74 of plate 38. When assembled, inner rod 70 will be disposed distal of pin 66.

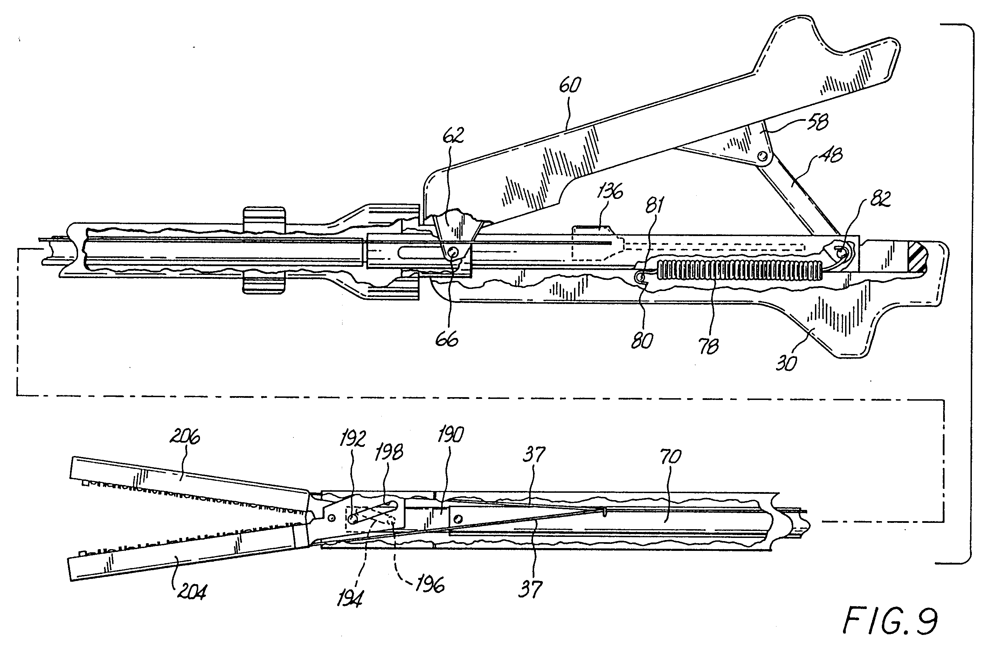

In operation, when movable handle 60 is urged towards stationary handle 30, the distal end of movable handle 60 pivots about pin 66, causing link 48 to urge plate 38 in a distal direction. When closed, movable handle 60 remains adjacent stationary handle 30 due to over centering of pin 54. Distal movement of plate 38, causes inner rod 70 to move distally as well. Spring 78, disposed adjacent plate 38 is secured to stationary handle protrusion 81 (see Fig. 9) by distal spring hook 80 and secured to cam pin 44 by proximal spring hook 82. Spring 78 serves to provide resistance to the closing of the movable handle and to facilitate the opening of the movable handle.

Turning to the endoscopic and distal portions of the surgical device of the present invention, with reference to Figures 1, 2 and 3, the endoscopic portion includes cover tube 102 (shown in two halves), inner rod 70, firing wires 37, articulation rod 96, and optional inner rod sheath 200. Proximal rotation knob 76 has an irregular outer surface to facilitate grasping and serves to allow the surgeon to rotate cover tube 102. Rotation knob 76 has inner circular disks 106 and 108 which define circular channel 110. Flange 112 of cover tube 68 is received within channel 110 in a manner which permits cover tube 102 to rotate with respect to rear cover tube 68 and, therefore, handle assembly 12.

The articulating mechanism includes articulating knob 90, rod 96 and articulating joint 16. Rod 96 has distal end 100 and proximal hook 98. Distal end 100 of articulating rod 96 is secured to articulating portion 116 by pin 101. The proximal end of rod 96 terminates in hook 98 which passes through slot 104 of lower tube 102 and is received within recess 94 of articulating knob 90. Articulating knob 90 is assembled about tube 102 by joining protrusions 91 and corresponding recesses 92. Hook 98, secured to the articulating knob at recess 94 and being slidable within slot 104 of tube 102, permits movement of the articulating knob along longitudinal axis X-X of endoscopic portion 14. Articulating portion 116 of endoscopic portion 14 is joined to cover tube 102 at joint 16. Opposing pins 120 are received within pin openings 118 of articulating portion 116 and pin openings 119 formed in articulating joint members 114. By moving articulating knob 90 longitudinally, articulating rod 96 is also moved longitudinally, thereby causing articulating portion 116 to pivot about pins 120 of articulation joint 16 in a direction away from the longitudinal axis X-X of endoscopic portion 14.

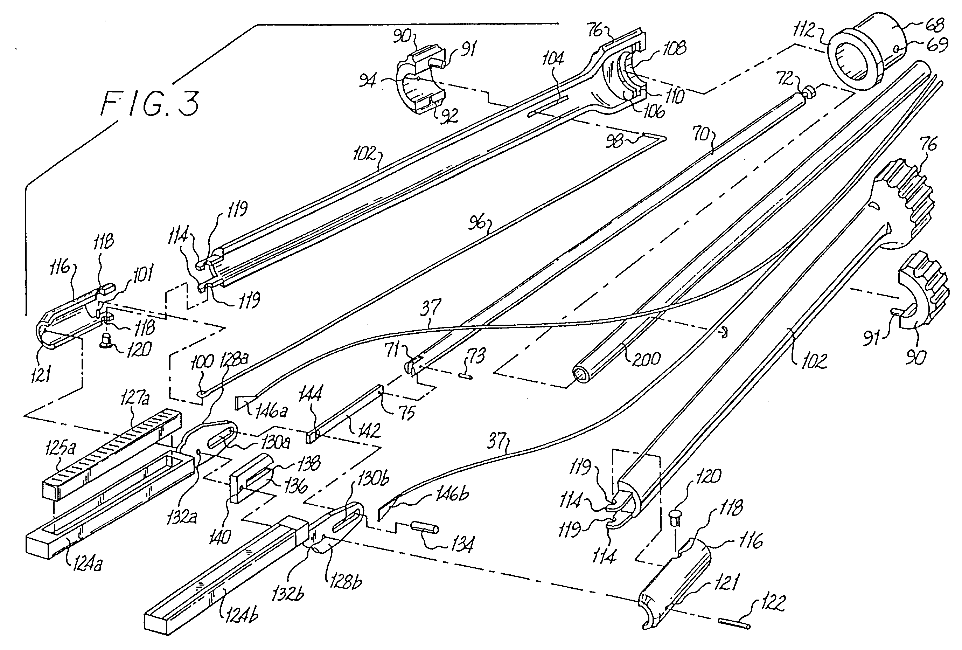

With reference to Figures 3, 6 and 7, fastener carrying cartridges 125a and 125b are disposed within jaws 124a and 124b and have tissue contacting surfaces 127a and 127b. Jaws 124 can be adapted to slidably receive cartridges 125 so as to provide means for reloading the instrument for multiple firings. Alternatively, the fasteners can be disposed directly into the jaws without the use of cartridges. Jaws 124 are secured to articulating portion 116 by pin 122. Pin 122 is received through pin openings 121 of articulating portion 116, pin openings 132a and 132b of jaw cam plates 128a and 128b and through pin opening 140 of jaw spacer 136. Jaw cam pin 134 passes through jaw cam slots 130a and 130b of jaw cam plates 128a and 128b, spacer slot 138 of jaw spacer 136 and pin opening 144 of articulating jaw cam plate 142. The proximal end of jaw cam plate 142 is secured to center rod 70 at recess 71 by pin 73 which passes through plate 142 at pin opening 75. Cam plate 142 is made of a flexible material to permit articulation and enable manipulation of the jaw camming mechanisms while the instrument is articulated.

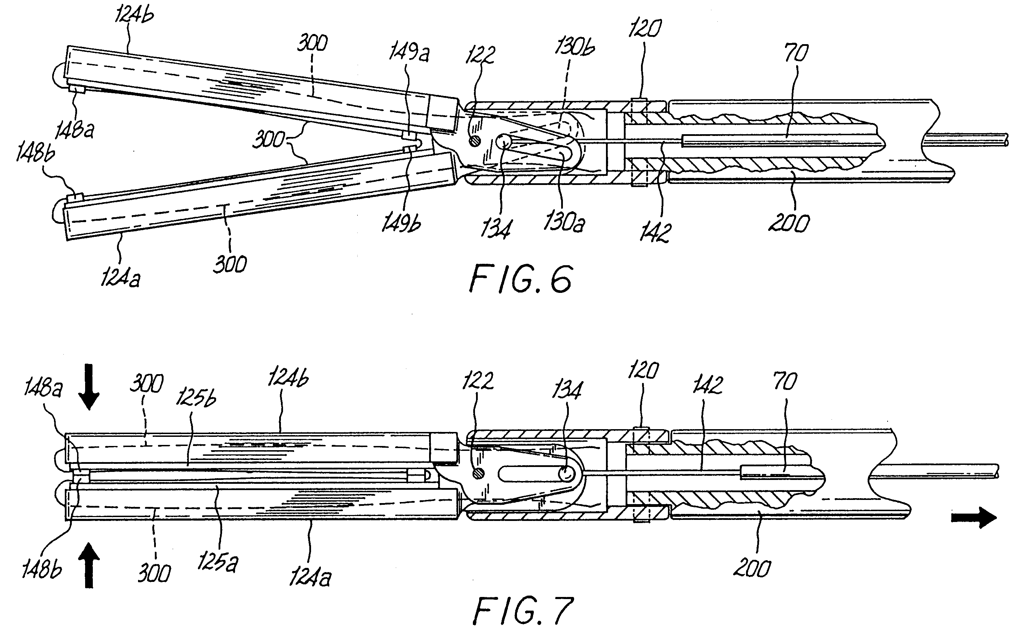

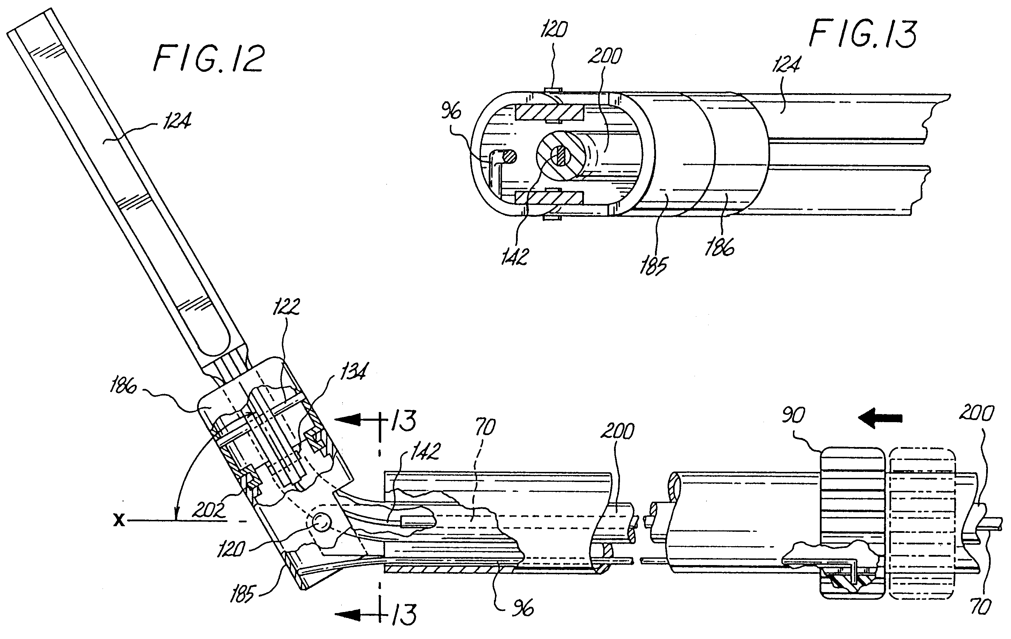

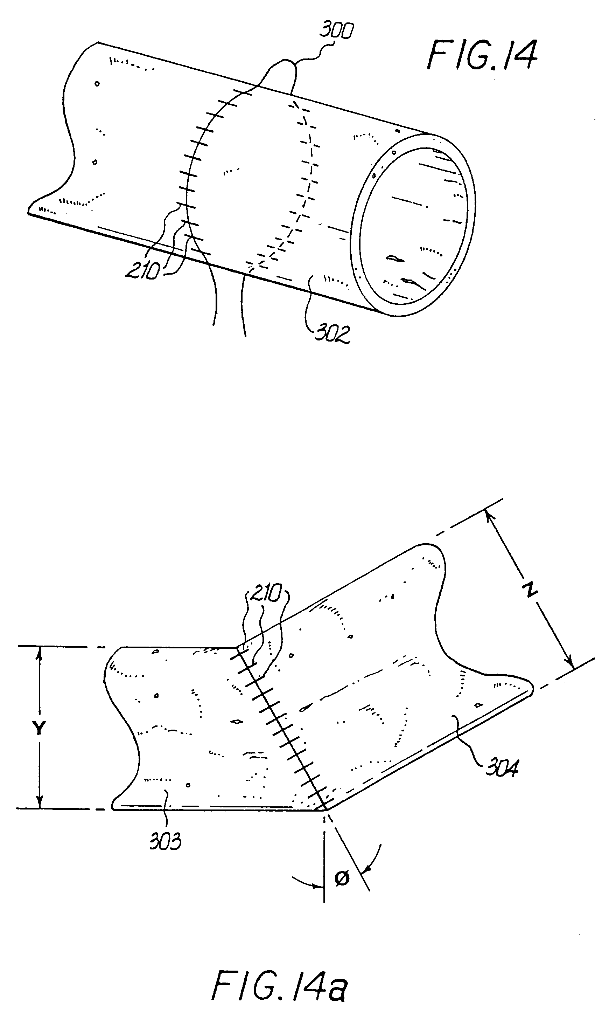

Firing wires 37 are also disposed in endoscopic portion 14 and have firing cams 146a and 146b which are disposed within jaws 124a and 124b, respectively. Suture 300 is positioned adjacent tissue contacting surfaces 127 and jaws 124 (see Figs. 6 and 7). Operation of the firing mechanism will be discussed in greater detail below. Optionally, inner rod sheath 200 can be provided to facilitate an alternate method of rotating the distal end of the instrument (see discussion of Figs. 12 and 13 below).

Turning to the jaw manipulation mechanism of the present invention, with reference to Figures 3, 4, 6 and 7, when the jaws are in the open position, jaw pin 134 is disposed at the distal end of slots 130a and 130b. In this position, inner rod 70 is in a forward or distal position and movable handle 60 is in its open position. By closing the handle assembly, i.e. moving movable handle 60 toward stationary handle 30, inner rod 70 and jaw cam pin 134 are drawn in a proximal direction. When jaw cam pin 134 travels through jaw cam slots 130a and 130b, jaws 124a and 124b are caused to pivot about jaw pin 122 and are urged towards each other. When the instrument is not articulated, the jaws will move towards the longitudinal axis X-X of the instrument. If the instrument is articulated, the jaws will move towards articulated jaw axis X'-X'. As shown in Figure 7, when the jaws are fully closed, jaw stops 148a, 148b, 149a and 149b are in contact and tissue contacting surfaces 127 of cartridges 125 are in substantial parallel alignment.

An alternative method for closing the jaws is illustrated in Fig. 9. In this embodiment, articulating cam plate 190 has pin 192 which travels in slot 198 of jaw 204. Jaw 206 has pin 196 which is configured to travel along slot 194 of cam plate 190. When movable handle 60 is urged towards stationary handle 30, inner rod 70 is drawn in a distal direction, causing cam plate 190 to move distally as well. As plate 190 moves distally, pin 192 causes jaw 204 to move towards jaw 206 and slot 194 causes pin 196 on jaw 206 to move jaw 206 towards jaw 204. When handle 60 is in a fully closed position, jaws 206 and 204 will have moved into substantial parallel alignment and be ready for firing.

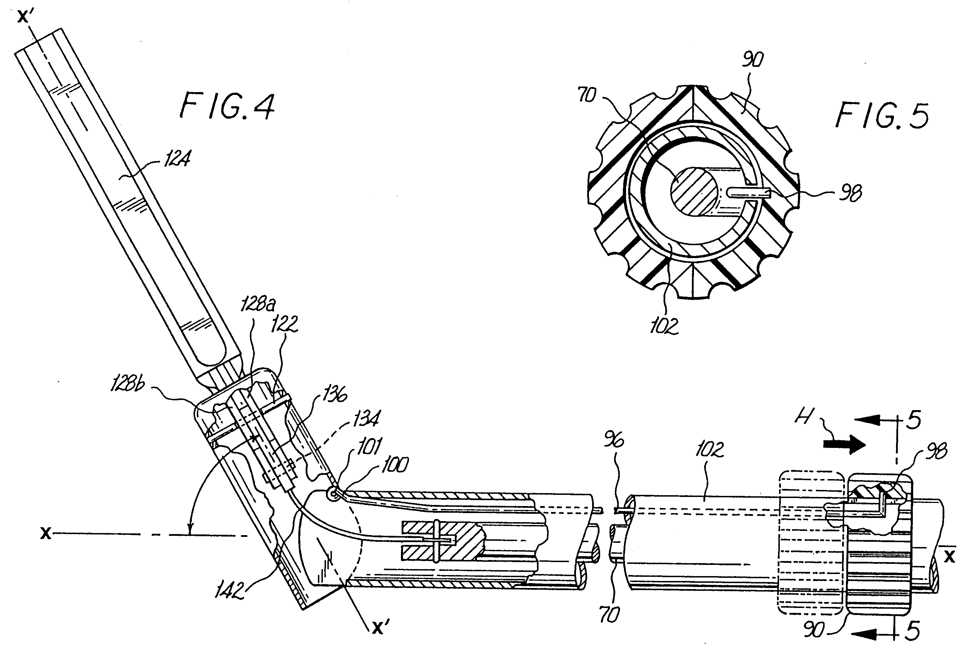

Referring to Figures 4 and 5, articulation is achieved by pulling articulating knob 90 in a proximal direction (arrow H). This proximal movement causes articulating rod 96 to pull on articulating portion 116 at pin 101. When articulated, axis X'-X' of jaws 124 and articulated portion 116 moves away from axis X-X of the non articulating endoscopic portion of the instrument. At any jaw position the distal end of the instrument can be articulated and the jaws will open and close towards longitudinal jaw axis X'-X' of the instrument.

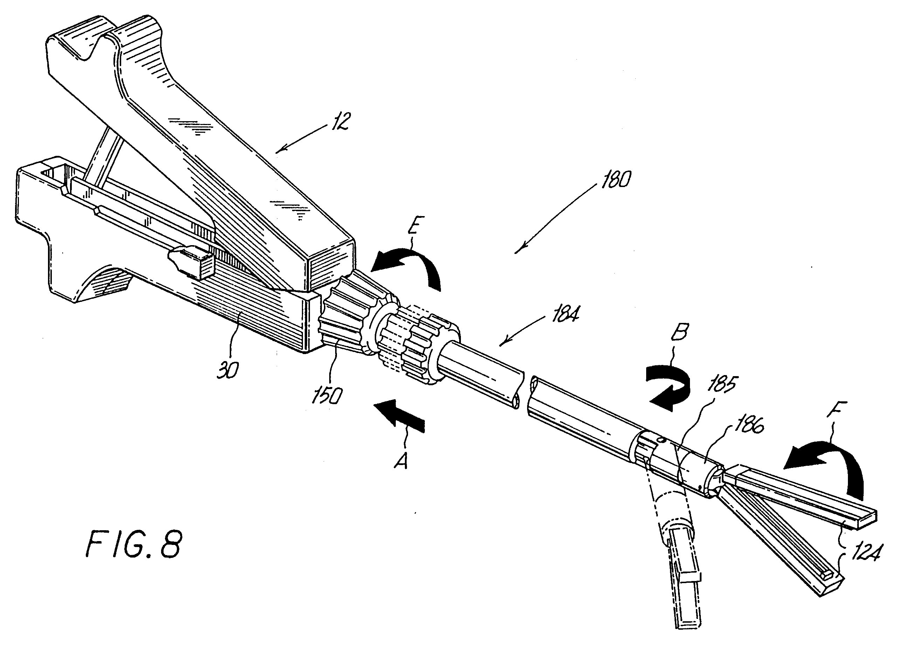

An alternative embodiment of the present invention is illustrated in Figs. 8, 12 and 13. In this embodiment, rotation knob 150 causes the rotation of endoscopic articulation portion 186 and jaws 124, illustrated by arrows E and F, respectively, relative to endoscopic portions 184 and 185. This embodiment includes center rod cover sheath 200 (see Fig. 3) which provides means to translate rotation movement from knob 150 to the distal portion of the instrument. Referring to Figs. 8 and 13, rotation of knob 150 causes sheath 200 to rotate, further causing distal articulating portion 186 and jaws 124 to rotate. Articulating portions 185 and 186 are rotatable with respect to each other by means of rotation joint 202. Both jaw cam pin 134 and jaw pivot pin 122 are located in rotatable articulation section 186. With sheath 200 secured to the proximal end of section 186, rotational movement bypasses the entire endoscopic portion proximal of joint 202. Figures 12 and 13 also show an alternative method of articulation. Here, distal movement of articulating knob 90 causes articulation rod 96 to push against portion 185, causing the distal end of the instrument to deflect away from axis X-X.

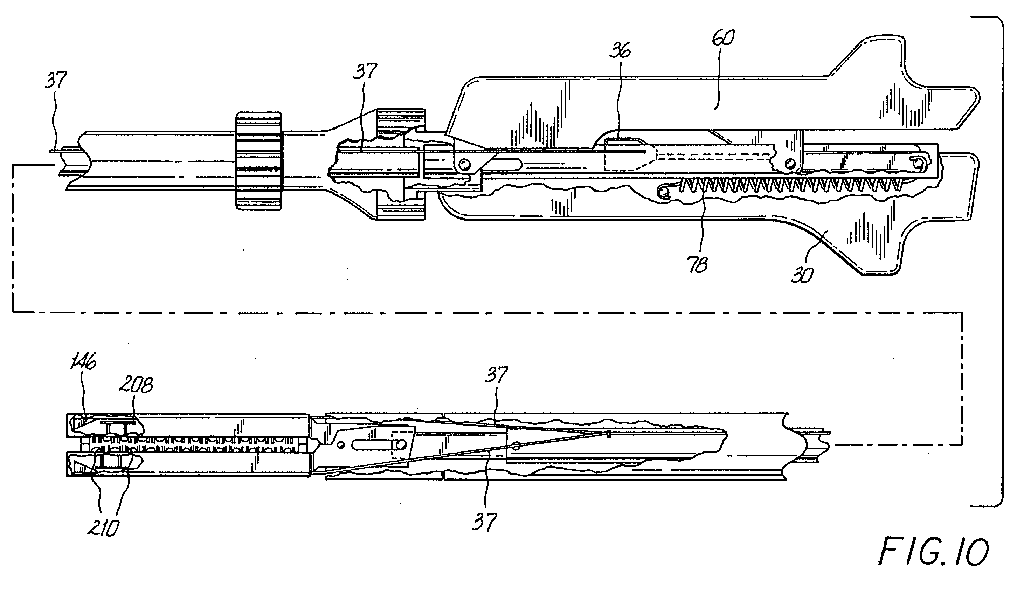

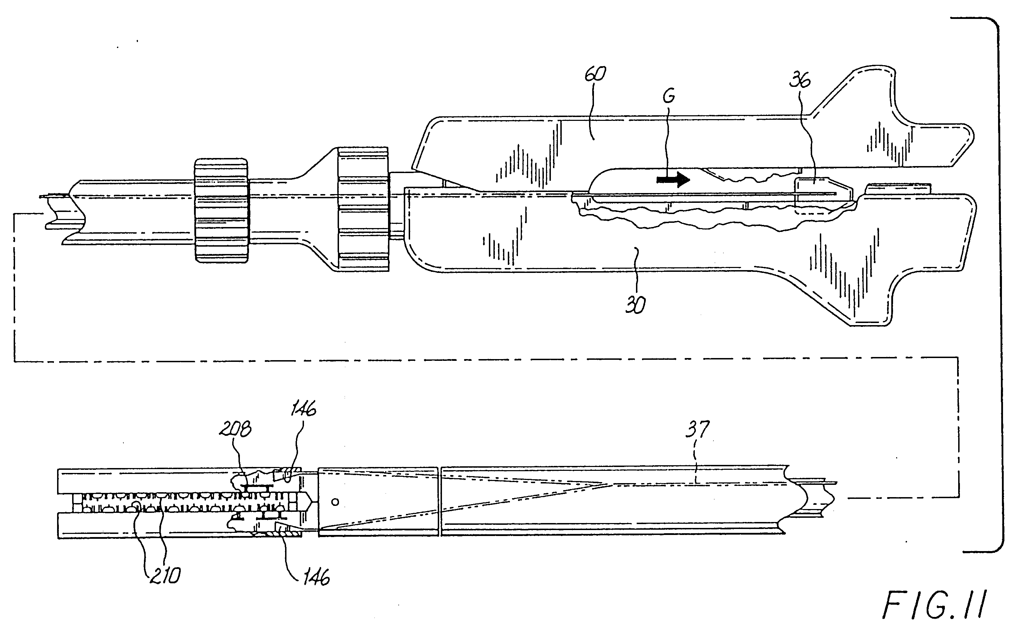

The firing mechanism of the present invention is best illustrated in Figs. 10 and 11. Once the movable handle 60 and stationary handle 30 are brought in the closed position, the firing mechanism can be actuated. Firing button 36 can be drawn in a proximal direction (arrow G) thereby causing firing wires 37 and attached staple pusher cams 146 to be drawn proximally. Proximal movement of cams 146 causes staple pushers 208 to sequentially fire staples 210 from the jaws. Providing separate means for firing staples 210 from each jaw, such as a firing button secured to each firing wire (not shown), is considered within the scope of the invention.

Staples 210 are slidably mounted within the jaws or jaw cartridges and deformed without the use of an anvil upon ejection therefrom as disclosed in U.S. Patent No. 4,821,939. Staples 210 are aligned such that their backspans are in substantial perpendicular alignment to the longitudinal axis X-X of the instrument when in the unarticulated position. As cams 146 are drawn across pushers 208, staples 210 are ejected towards axis X-X. It will be appreciated that when the instrument is in an articulated position, the staple backspans are in substantial perpendicular alignment to longitudinal axis X'-X' of the articulated jaws and are capable of being fired toward that longitudinal axis while articulated.

Referring to Figures 3, 6, 7 and 13, Suture 300 has two end portions, a body portion and is partially disposed adjacent tissue contacting surfaces 127 of fastener cartridges 125. Suture 300 is held in place by means of jaw stops 148 and 149 in a manner similar to that disclosed in U.S. Patent No. 4,821,939. As shown in Figures 6 and 7, suture 300 loops back along jaws 124 with the two end portions terminating proximal of jaws 124. When applied, suture 300 is secured between the backspans of fasteners 210 (See figs. 10 and 11) and the tissue or organ, **such as, for example, intestines or blood vessels 302,** to which the fasteners are applied (See Fig. 14). Any suture placement which allows the suture to be secured to body tissue by fasteners is considered to be within the scope of the present invention as defined in the appended claims.

In operation, the instrument of the present invention is inserted through cannula with the handles and jaws in the closed position. Once within the abdominal cavity, movable handle 60 may be moved away from stationary handle 30, aided by spring 78, thereby causing center rod 70 to move in a distal direction, causing the jaws to open. Using the articulating and rotating mechanisms if desired, the organ or tissue to which the purse string is to be applied will be positioned between the jaws. The jaws may then be closed by bringing movable handle 60 towards stationary handle 30. With the tissue or organ disposed adjacent tissue contacting surfaces 125, the surgeon may then slide firing button 36 proximally to eject the staples into the tissue, thereby securing the suture. After firing, the surgeon will then open the handles and jaws as previously described to release the organ or tissue. Finally, by closing the handles and jaws, the surgeon may remove the instrument through the cannula.

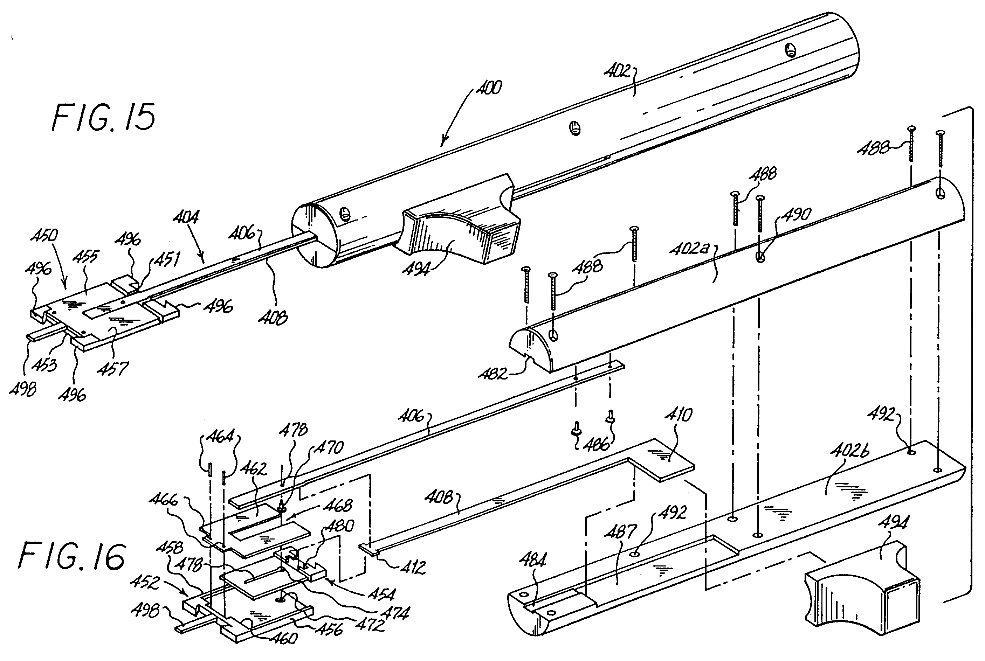

Preferably, with reference to Figs. 15-18 and 21-25, a stent is provided to facilitate approximation of tubular organs and/or to facilitate the joining of tubular organs by means of fasteners. In, Figs. 15-18, an expandable stent 450 is disposed at the distal end of elongate member 404. Generally, expandable stent 450 has first side 455 and second side 457 which are transverse to proximal surface 451 and distal surface 453.

With reference to Fig. 16, expandable stent 450 includes a stationary member 452 and a movable member 454. Stationary member 452 includes opposing, raised side walls 456 and 458 and raised distal wall 460. Stationary cover plate 462 is secured to stationary stent member 452 by pins 464 which pass through holes 466 in cover plate 462 and corresponding holes (not shown) in stationary stent member 452. Movable stent member 454 is received between cover plate 462 and stationary stent member 452 and slidable therebetween.

Elongate member 404 has two adjacent portions 406 and 408. Stationary elongate portion 406 is received in slot 468 of cover plate 462 and is secured to stationary stent member 452 by means of screw 470 which passes through screw holes 472, 474, and 476. Movable elongate member 408 has T-shaped portion 412 at its distal end and right angle projection 410 at its proximal end. T-shaped portion 412 is configured and dimensioned to be received in recess 480 in the proximal end of movable stent 454. Both elongate members 406 and 408 are received in slots 482 and 484, respectively, in handle portion 402. Stationary elongate member 406 is secured to hemi portion 402A by means of screws 486 while movable elongate portion 408 is disposed in handle portion 402 in slots 484 and wide slot 486. Slider knob 494 further secures elongate portion 408 by receiving a portion of right angle portion 410 therein. As shown, screws 488 secure hemi portion 402A to hemi portion to 402B by passing through slots 490 and 492.

Returning to the stent portion, preferably both movable stent portion 454 and stationary stent portion 452 have inwardly facing projections 496. These projections facilitate placement of a tubular organ over the stent as will be described in greater detail below. Additionally, optional stent projection 498 protrudes from the distal portion of stationary stent 452, the function of which is also described below.

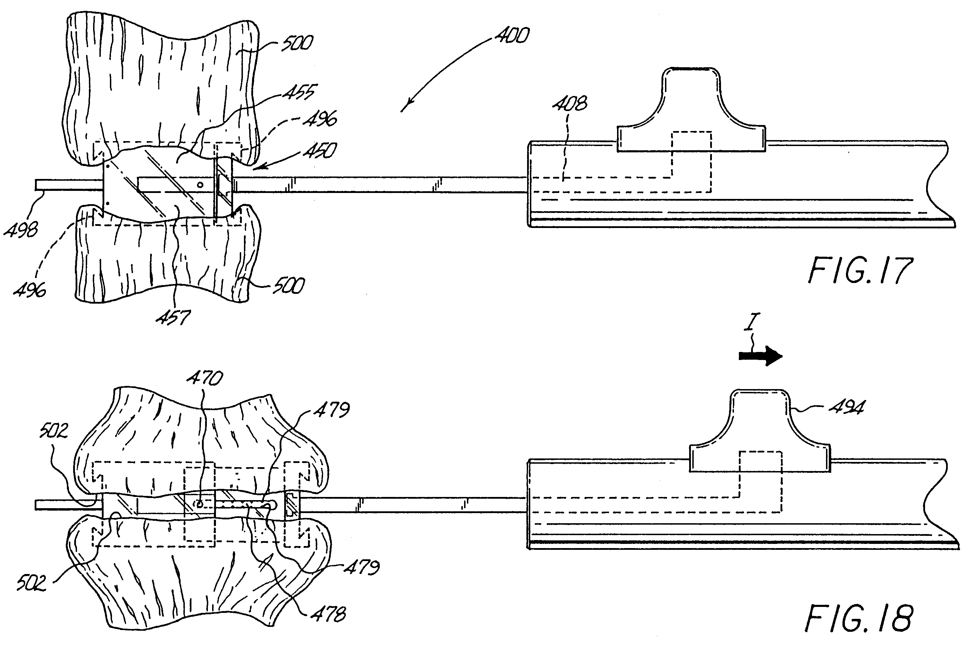

In operation, with reference to Figs. 17 and 18, stent applicating device 400 is shown with two portions of tubular organs disposed over stent 450. In Fig. 17, stent 450 is in its collapsed position. In this state, movable elongate member 408 is in its distal most position, which facilitates placement of tubular organs 500 over projections 496. When placing the organs over sides 455 and 457 of stent 450, projection 498 inhibits over placement of the organs, thereby aiding in retaining each organ on its respective side of the stent. After organs 500 are placed on each side of the stent, knob 494 is moved proximally (arrow I) to slide movable elongate member 408 relative to stationary member 406, thereby causing movable stent portions 454 to slide proximally relative to stationary stent member 452. As shown, slot 478 in movable stent member 454 permits movement of movable stent 454 relative to screw 470. Slot 478 can have inwardly projecting serrations 479 to facilitate incremental expansion of stent 450.

Upon expansion of stent 450, tubular organs 500 are caused to stretch and flatten out along the longitudinal span of stent. Projections 496 facilitate in preventing organs 500 from slipping off stent 450. Also, upon expansion of stent 450, edges 502 of each organ tend to move toward the longitudinal axis of the instrument, thereby decreasing the distance between the two organs to be joined. Once the tubular organs are disposed about a stent in this manner, various methods of joining the organs can be employed which will be described hereinbelow.

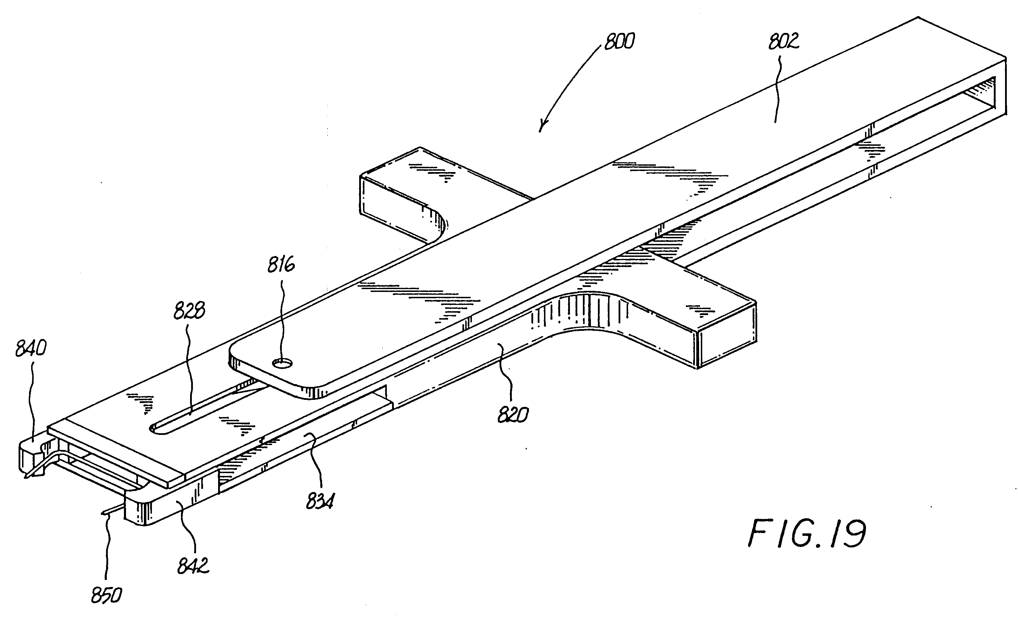

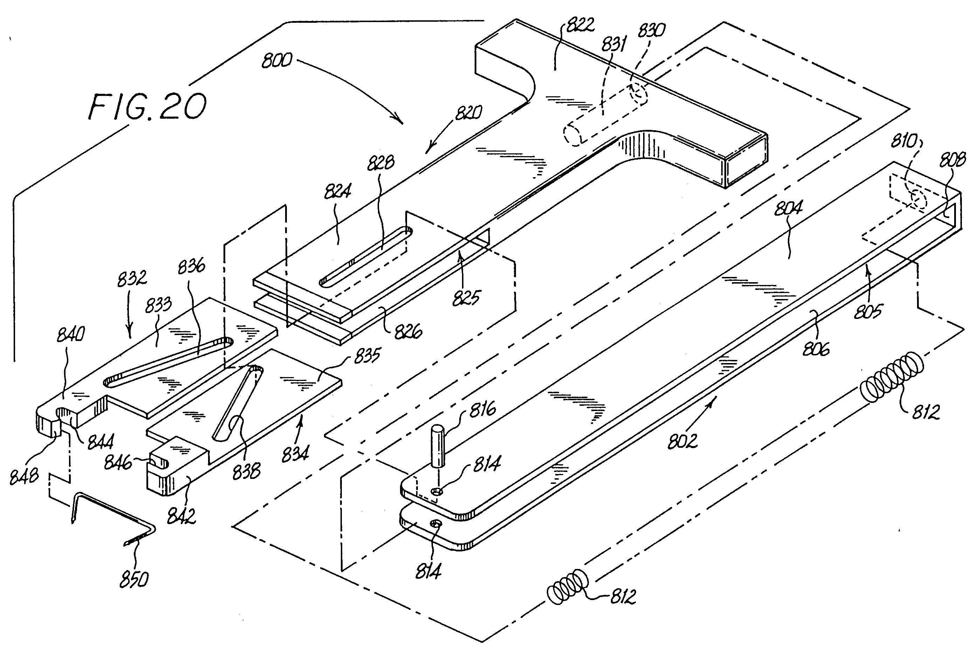

A single shot staple applier not in accordance with the invention is illustrated in Figs. 19-22. Stapler 800 includes movable housing 802 having elongate portions 804 and 806 extending distally from vertical wall 808. Elongate members 804 and 806 define slot 805 therebetween in which stationary housing 820 is slidably received. Stationary housing 820 includes proximal end 822, which is generally configured in a T-shape to facilitate gripping by the user. The distal portion of housing 820 includes elongate members 824 and 826 which define slot 825 therebetween. Staple deforming assemblies 832 and 834 are received in slot 825 and are configured and dimensioned to reciprocate laterally therein. The staple deforming assemblies include flat portions 833 and 835 having slanted slots 836 and 838, respectively. Means for engaging a staple are shown in Fig. 20, wherein staple engaging member 842 generally projects upward from the distal end of flat portion 835 while engaging member 840 projects generally downward from flat portion 833. This upward/downward configuration permits flat portions 833 and 835 to overlap during use while allowing staple engaging members 840 and 842 to approximate in a common plane. The staple engaging members further include staple receiving and deforming slots 844 and 846 which are configured and dimensioned to receive staple 850. Preferably, each staple deforming slot has a groove 848 to facilitate placement and control the staple during use.

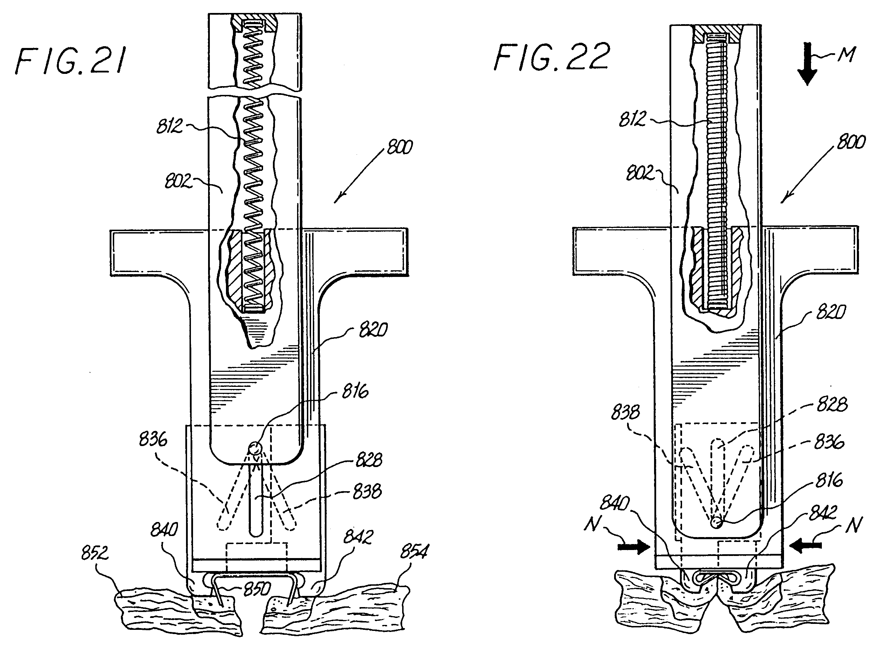

When assembled, stationary housing 820 is slidably received in slot 805 of movable housing 802. Spring 812 is disposed between vertical wall 808 of movable housing 802 and passes through orifice 830 to enter bore 831 in the proximal end 822 of stationary housing 820. Pin 816 passes through orifices 814 in elongate members 805 and 806, slot 828 in elongate member 824 (and a corresponding slot in elongate member 826 - not shown) and slanted slots 836 and 838. A staple can be loaded between engaging members 840 and 842 when pin 816 and movable housing 802 are proximally positioned (Fig. 21) with respect to stationary housing 820.

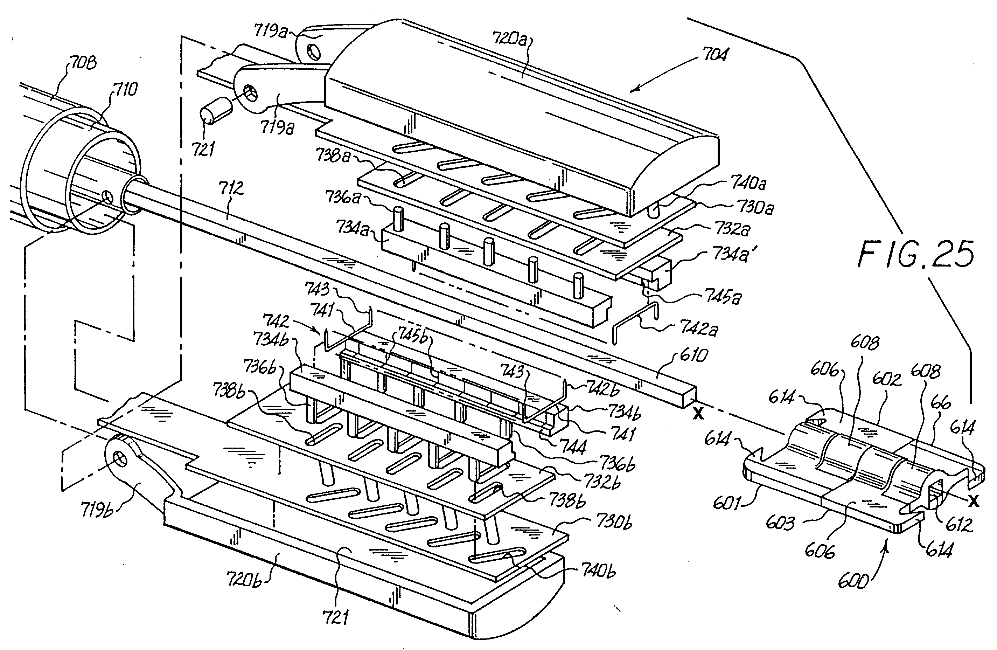

With reference to Figs. 21 and 22, single shot stapler 800 is shown applying a staple across two tubular organs. With tubular organs 852 and 854 properly approximated, i.e., by hand manipulation or through the use of a stent (see stents in Figs. 17 and 25) the distal end of the staple applier 800 is brought in to contact with both ends of the tubular organ. By sliding movable housing 802 relative to stationary housing 820, pin 816 is caused to travel in a distal direction through slots 828, 836 and 838. As shown in Fig. 22, this movement of pin 816 causes staple deforming assemblies 832 and 834 to overlap and further cause staple engaging members 840 and 842 to close staple 850 into a pretzel-shape. After firing, pin 816 can be moved in a proximal direction to cause staple engaging members 840 and 842 to release staple 850, leaving the ends of tubular organs 852 and 854 joined. Subsequent application of additional staples can complete the anastomosis of the tubular organs. While stapler 800 can be used to perform an anastomosis without further instrumentation, preferably a stent is used to facilitate orientation of the tubular organs.

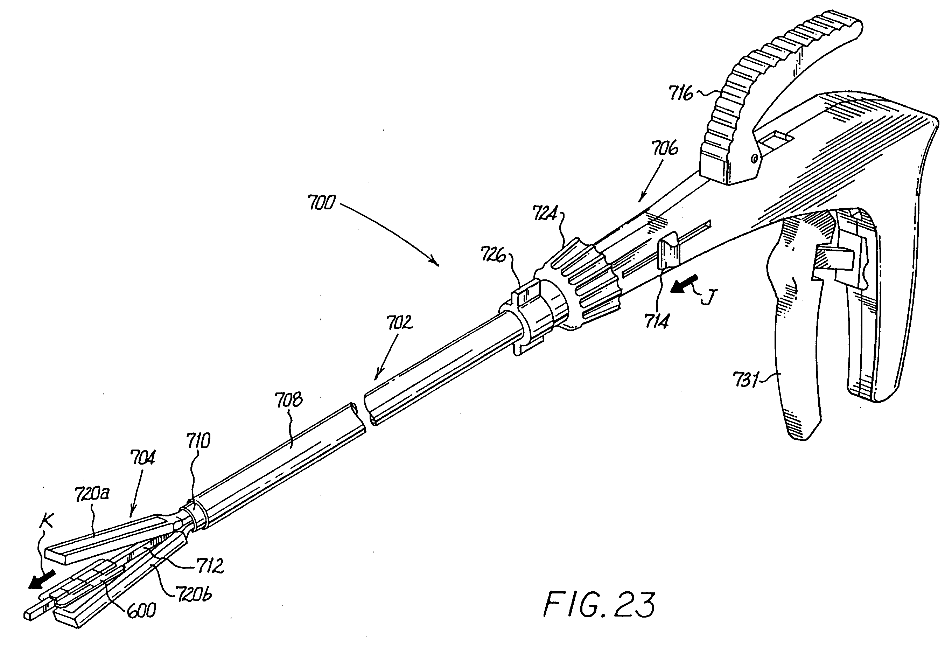

An alternative embodiment of a stent member is shown in Figs. 23-29. Turning to Fig. 25, fragmentable stent 600 includes a plurality of interrelating stent portions 601, 602, 603 and 604. Each of these stent portions have a generally planar portion 606 integral with a generally curved portion 608. The generally curved portions are arranged such that they are aligned along longitudinal axis X-X of the instrument and such that generally planar portions 606 are disposed on either side of longitudinal axis X-X. Stent rod 610 passes through apertures 612 of curved portions 608 of each stent portion. Stent rod 610 is designed to engage the stent portions so as to prevent their rotation relative to axis X-X and to at least partially maintain and control the longitudinal orientation of the stent with respect to the stapling portion of the instrument.

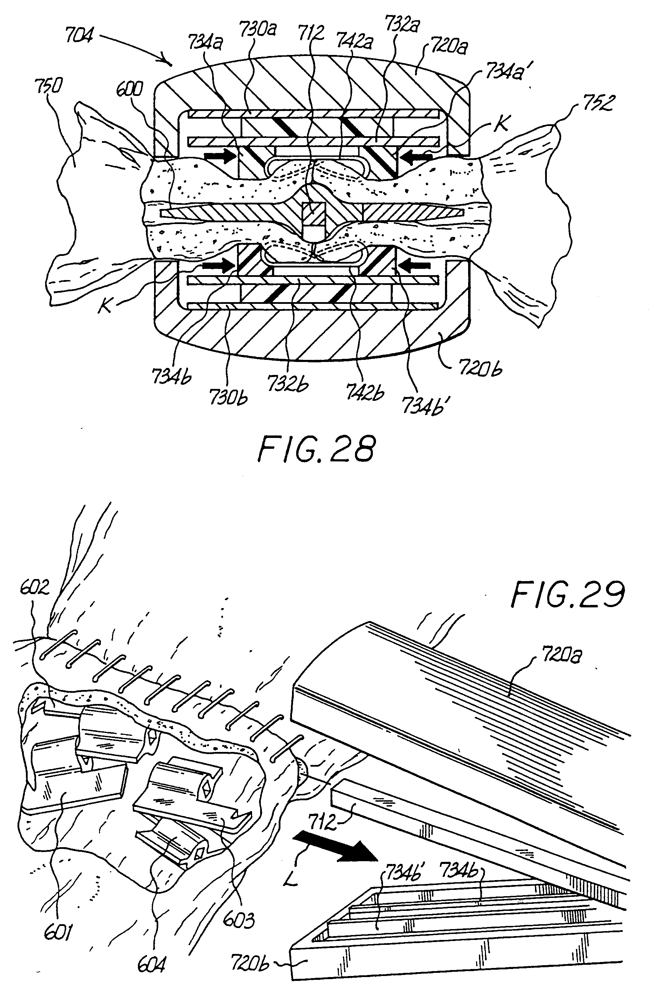

Preferably, stent 600 includes projections 614 which are angled toward curved stent portions 608 and generally lie in a plane which cuts through axis X-X. Projection 614 function in a manner similar to projections 496 in expandable stent embodiment 450 (see e.g., Fig. 15). Stent portions 601-604 are designed to be freely movable from each other when stent rod 610 is removed from orifices 612. The purpose of this configuration is described in greater detail, below, in conjunction with Fig. 29. It is understood that several variations of stent 600 are considered within the slope of the present invention. Rod 610, for example, need not pass through all stent members. Furthermore, for example, stent 600 could be entirely or partially planer, i.e., curved portions 608 are preferred but optional.

With reference to Figs. 23-29, a novel surgical stapler 700 is shown. Stapler 700 includes endoscopic portion 702 having proximal and distal ends, distal stapling portion 704 and proximal handle portion 706. Endoscopic portion 702 and stapling portion 704 are configured and dimensioned to be insertable into a cannula, with theludes outer tube 708, inner tube 710 and stent rod 712 passing therethrough. Stent rod 712 and inner tube 710 are manipulable by knob 714 and lever 716 respectively. If surgical stapler 700 is used during endoscopic or laparoscopic procedures where positive gas pressure exists in a body cavity, a seal 703 is preferably provided within endoscopic portion 702 to prevent or reduce the flow of gases therethrough. As is known in the art, a seal 703 is provided with suitable orifice to permit passage of structures through endoscopic portion 702. Suitable seal materials include synthetic foams and rubbers.

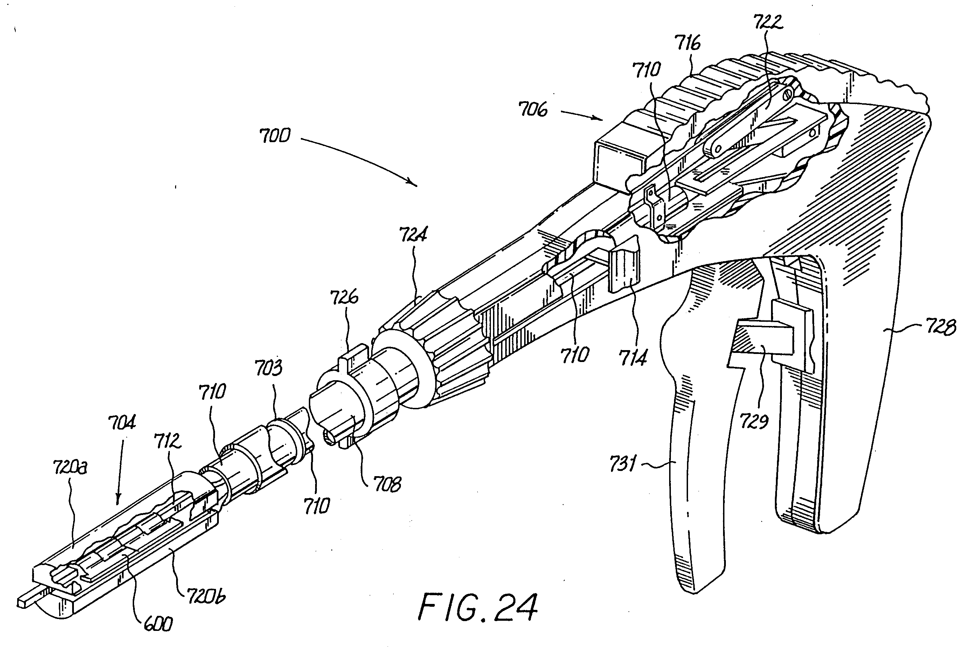

With reference to Figs. 23 and 24 where the articulation joint has not been depicted, stapling portion 704 is disposed at the distal end of endoscopic portion 702 and includes jaws 720A and 720B shown in the open position. With the jaws open, stent rod knob 714 is slidable relative to the longitudinal axis of the instrument to manipulate the orientation of stent 600 relative to distal jaw portion 704. As shown in Fig. 24, when stent rod 712 is sufficiently retracted in the proximal direction, jaws 720A and 720B can be properly closed about stent 600. Closure of the jaws is accomplished by causing inner tube 710 to move distally relative to jaws 720A and 720B, thereby causing the distal end of tube 710 to contact the proximal ends of the jaws. This contact causes the jaws to pivot toward each other and close. Means for causing inner tube 710 to move relative to the proximal ends of jaws of 720A and 720B are disposed in proximal handle assembly 706. As shown in Fig. 24, movement of lever 716 towards handle assembly 706 causes linkages 722 to move inner tube 710 in a distal direction. A similar closure mechanism is disclosed in commonly assigned U.S. Patent No. 5,040,715 wherein an anvil member is cammed towards a staple carrying member by means of relative movement with respect to an inner tube. It is to be understood that jaw approximation by means of manipulating an inner tube is but one method of closure and that those skilled in the art could devise other suitable means for closing jaws 720A and 720B, such means are considered to be within the scope of the present invention. For example, closure by means of a cam pin disposed in cam slots in the proximal ends of the jaws, as disclosed in Fig. 3, is also a suitable means for closing the jaws of stapler 700.

Stapler 700 further includes means for rotating endoscopic portion 702 and jaw assembly 704 relative to handle assembly 706. Rotation knob 724 is disposed about endoscopic portion 702 and may be integral with outer tube 708 (similar to rotation portion 76, shown in Fig. 3). Locking member 726 provides means for locking endoscopic portion 702 relative to handle 706. A detailed description of a similar locking mechanism is disclosed in commonly assigned U.S. Patent No. 4,728,028.

Handle portion 706 includes rear handle 728, trigger 731 and safety 729 disposed therebetween. After properly positioning stapling portion 704 about organs to be joined, safety 729 can be deflected to permit movement of trigger 731. Trigger 731 functions to eject staples from the instrument which will be described in greater detail below.

Turning to stapling assembly 704, with reference to Fig. 25, jaws 720A and 720B house similar, opposing staple carrying and firing elements. While stapling assembly 704 will be described with reference to lower jaw 720B, jaw 720A has substantially identical components facing in an opposite sense. The staple firing mechanism generally includes slidable cam plate 730B, stationary cam plate 732B and staple deforming bars 734B and 734B'. Staple bars 734B and 734B' have cam posts 736B which are slidably received in lateral slots 738B in cam plate 732B and through slanted slots 740B in cam plate 730B. Cam plate 730B and 732B are received in recess 721 in lower jaw 720B. Slidable cam plate 730B is operably associated with trigger 731 such that movement of the trigger causes plate 730B to move relative to plate 732B. Means for connecting plate 730B to trigger 731 include cable, wire, tubular structures and the like.

Staples 742B are disposed between staple deforming bars 734B and 734B' which have shoulders 744 and slots 745B to facilitate placement and storage of staples 742B therein. Staples 742B are disposed with backspans 741 perpendicular to axis X-X of the instrument (similar to the staple orientation in Figure 11), and have prongs 743 oriented towards jaw 720A. While two staples are shown with reference to jaw 720B, obviously, any number of staples suitable for performing a surgical procedure can be disposed between the staple forming members 734B.

In order to deform staples 742B to a closed configuration, slidable cam plate 730B is urged in a proximal direction. Proximal movement of plate 730B causes cam posts 736B to travel along slots 740B and 738B, thereby causing staple deforming bars 734B and 734B' to slide laterally. Lateral movement of both staple deforming bars causes staples 742B to deform to a closed configuration.

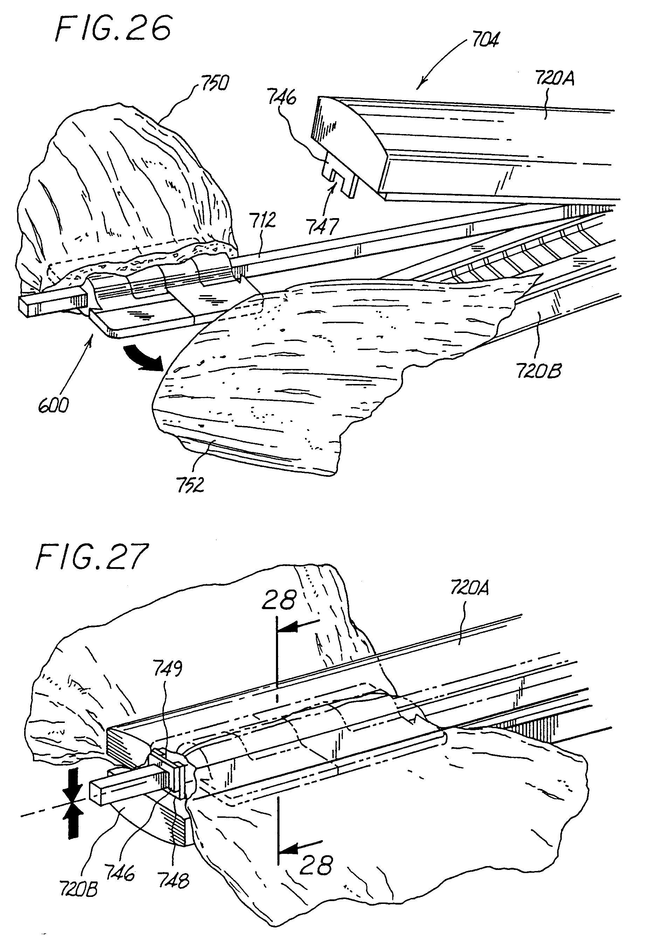

Use of the novel stapling device in performing an end to end anastomosis procedure is shown in Figures 26-29. In operation, stent 600 is positioned distally, away from jaw assembly 704 by advancing stent rod 712 in a distal direction. Tubular organ sections 750 and 752 are then positioned over the flat portions of stent 600. As shown in Figs. 26 and 27, stent rod 712 extends past stent 600. This extension facilitates preventing over placement of the tubular organ sections. Though not shown in this embodiment, stapling portion 704 can be fabricated to articulate with respect to endoscopic portion 702, in a manner similar to that disclosed above, to facilitate placement of tissue. After the tubular organs have been placed over stent member 600, stent 600 and rod 712 can be retracted by sliding knob 714 (Fig. 23) to position tubular organs 750 and 752 between jaws 720A and 720B. Jaws 720A and 720B can then be closed, i.e., by causing inner tube 710 to move distally. Distal movement of inner tube 710 causes the distal end of tube 710 to cam against the proximal end portions 719A and 719B of the jaws (Fig. 25) which further causes jaw portions 720A and 720B to pivot about pin 721. Alignment means for ensuring proper orientation of the stent rod and stent relative to the jaw assembly can also be provided. As shown in Figs. 26 and 27, alignment projections 746 and 748 have recesses 747 and 749, respectively, which are configured and dimensioned to receive the distal end of stent rod 712.

To fire the instrument, safety 729 (Fig. 24) is disengaged and trigger 731 is pulled in a proximal direction, thereby causing movable cam plates 730A and 730B to move in a proximal direction, as described above. With reference to Fig. 28, a cross sectional view of distal stapling assembly 704 is shown after firing. Arrows K depict lateral movement of staple pushing members 734 which have moved inward to deform staples 742 into a pretzel-shaped configuration, thereby joining tubular organs 750 and 752.

With reference to Fig. 29, after firing the instrument jaws 720A and 720B are opened and moved proximally in the direction of arrow L, away from the tubular organs. Withdrawal of the instrument causes stent rod 712 to be withdrawn from orifices 612 of the fragmentable stent, thereby permitting the stent to fragment within the joined organs. Alternatively, stent rod 712 can be withdrawn prior to firing the staples. Stent portion 601-604 may then pass through the body.

While surgical stapler 700 has been shown in conjunction with the use of a stent and stent rod, the instrument can be used without a stent. With reference to Fig. 28, for example, when jaws 720A and 720B are closed about tubular organs, tissue can be forced between prongs 743 of staples 742. Upon firing of the instrument, staples 742 are closed in such a manner as to engage and approximate the tubular organs. With reference to Figure 14a, if the tubular tissue sections to be joined are of different diameter, the smaller diameter tissue 303 (having diameter Y) can be cut at an angle φ so that the angled cut line is dimensioned closer to diameter Z of tubular tissue 304. With angled tubular tissue 303 approximated and juxtaposed to tubular tissue 304, the two tissues can be joined by fasteners, such as, for example, staples ejected from the surgical stapling instrument disclosed herein.

Expandable stent 450 (described above) and fragmentable stent 600 can be fabricated from any suitable bio-compatible material. Preferably, absorbable materials, such as those comprising glycolide and lactide, are used. Most preferred are stents made from materials which soften over time when exposed to body tissue and fluids. Stents made from polyvinyl alcohol (PVA), for example, are sufficiently rigid to permit expansion and approximation of tissue, yet soften over time to permit safe passage of the stent after the tissue has been joined, e.g., by stapling. Suitable plasticized PVA resins are available from Air Products and Chemicals, Inc., Allentown, PA under the VINEX® trademark. VINEX® 2025, for example, can be extruded into a film and compression molded to obtain a stent part. Alternately, PVA can be plastized with water, converted into film by compression molding and then compression molded into a stent part.

The following examples relate to the preparation of suitable stents.

### EXAMPLE I

### Preparation of stent using VINEX® 2025 plasticized PVA film

1. Provide a mold configured to the dimensions of the stent (see step 3).
2. Spray a light, thin film of heat-stable silicone release agent on parts of the mold to which the PVA will be exposed. Remove any excess.
3. Using scissors, carefully cut a piece of PVA film (VINEX® 2025 plasticozed PVA film extruded to a thickness of about 0.045") with dimensions 1 1/2" x 3/4". Place the film on the cavity insert of the mold. The cavity insert has dimensions 1/ 5/16" x 5/8". Put the mold together and place in a platen press.
4. Set the platen temperature to 150_C.
5. Close the plates of the oven and set the kiss pressure at either 0 or 70 bar (0 or 1000 lbs./sq. inch). Set the time at approximately 25 minutes. The duration must be long enough such that the plates reach 150_C. during the kiss.
6. Set the cure pressure at 2800 bar (40,000 lbs./sq. inch) for four minutes. Set the mist for five minutes and the cool for ten minutes.
7. The press will open when the cool has finished. Take the molded part out of the mold and place in a dry room.

### EXAMPLE II

### Preparation of PVA film and stent

1.0 Preparation of Film
1.1 Weigh out 7.5±0.5 gm PVA (7800 molecular weight, 88% hydrolyzed).
1.2 Weigh out 3.75±0.25 gm warm pyrogen free water.
1.3 Add water to PVA. Add approximately one ml and mix vigorously. Continue to add one ml at a time and mix until all water is used.
1.4 Roll into a tight ball using your hands.
1.5 Place the ball on top of a teflon-coated aluminum plate in a platen press with 0,064 cm (0.025") feeler gauges alongside the ball. Place another teflon coated aluminum plate on top of the ball.
1.6 Set the press close delay to 0.1 hours and vacuum to 0.2 hours while setting the plate temperature to 170_C.
1.7 Set the kiss to approximately 15 minutes. The kiss duration should be long enough to allow the temperature to reach 170_C and hold there for two minutes. The pressure should be 70 bar (1000 lbs./sq. inch).
1.8 Set the cure to five minutes with pressure at 2100 bar (30,000 lb./sq. inch).
1.9 Set the mist to five minutes and cool to six minutes.
1.10 The press will open after the cool is completed. The teflon-coated plates should come apart easily. The film should look clear and should be placed under vacuum for 18 hours at approximately 60_C.
2.0 Preparation of Stent
2.1 Spray a light, thin film of heat-stable silicone release agent on the parts of the mold to which the PVA will be exposed. Remove any excess.
2.2 Using scissors, carefully cut a piece of the PVA film with dimensions 3,8 cm x 1,9 cm (1 1/2" x 3/4"). Place the PVA film on the cavity insert. The cavity insert has dimensions 3,3 cm x 1,6 cm (1 5/16" x 5/8"). Put the mold together and place in the platen press.
2.3 Set the platen temperatures at 175_C.
2.4 Close the plates of the oven and set the kiss pressure at either 0 or 70 bar (0 or 1000 lbs./sq. inch). Set the time at approximately 25 minutes. The time must be long enough such that the plates reach 175_C and hold for two minutes.
2.5 Set the cure pressure at 2800 bar (40,000 lbs./sq.inch) for four minutes. Set the mist for five minutes and the cool for ten minutes.
2.6 The press will open when the cool has finished. Take the molded part out of the mold and place the part in a dry room.

The surgical stapling devices 10 and 700 are for endoscopic use, for end-to-end and side-to-side anastomosis of tubular organs such as intestines, arteries and the like.

The claims which follow identify embodiments of the invention additional to those described in detail above.

## Claims

1. A surgical instrument (10) for the anvil-less stapling of a purse string to tissue, the instrument comprising:
an elongate portion (14) having proximal (12) and distal (20) end portions and a longitudinal axis:
a pair of jaws (124), each extending on a longitudinal jaw axis from said distal end portion; and
a plurality of fasteners (210) disposed within each said jaws, the instrument being characterised in that:
i. the instrument is suitable for endoscopic surgery;
ii. the elongated portion (14) is articulated at an articulation joint (16) such that said distal end (20) of the elongated portion and said pair of jaws (124) can deflect at joint (16) away from said longitudinal axis.

2. The instrument of claim 1, further comprising at least one suture (300) at least partially disposed adjacent said pair of jaws.

3. The instrument of claim 1 or 2, further comprising means (16) for articulating said pair of jaws relative to the longitudinal axis of said endoscopic portion, the longitudinal jaw axis of each said jaw being substantially parallel to said endoscopic longitudinal axis when the instrument is in an unarticulated position.

4. The instrument of claim 3, wherein when said instrument is articulated, said longitudinal jaw axis is positioned at an angle with respect to the longitudinal axis of said endoscopic portion.

5. The instrument of any of the preceding claims, further comprising a stent (450) extending from said elongate portion distal end (20), said stent comprising a first member (452) and a second member (454) each having proximal and distal ends, wherein said first and second members are longitudinally slidable relative to each other such that the longitudinal distance between said first member distal end and said second member proximal end is adjustable.

6. The instrument of any of the preceding claims, further comprising a stent (600) extending from said elongate portion distal end, said stent comprising a plurality (601-4) of interrelating stent portions, each said portions having a generally planer portion (606) integral with a generally curved portion (608), said stent portions being arranged such that said generally curved portions are aligned along a longitudinal axis and at least one said generally planer portions is disposed on one side of said longitudinal axis and at least one said generally planer portions is disposed on the other side of said longitudinal axis.

7. The instrument of claim 6, wherein each said generally curved portions have a longitudinal aperture (612) adapted to receive an elongate stent rod therethrough.

8. The instrument according to claim 5, 6 or 7, wherein the stent is at least partially fabricated from a bioabsorbable material.

9. The instrument of any of the preceding claims further comprising means for deforming said fasteners.

10. The instrument of any of the preceding claims, wherein at least one said jaws has a stationary plate member (732) and a movable plate member (730) said movable plate member being longitudinally slidable within said jaw, each said plate members having a plurality of slots (738, 740) passing therethrough;
a pair of bars (734) longitudinally disposed within said jaw, said bars having projections adapted to pass at least partially through said plurality of plate member slots, wherein said plurality of fasteners are disposed between said bars and movement of said movable plate member causes each said bars to move laterally towards each other, thereby simultaneously deforming said fasteners.

## Patentansprüche

1. Chirurgisches Instrument (10) zum anschlaglosen Verklammern einer zusammenziehbaren Naht an Gewebe, wobei das Instrument umfaßt:
einen langgestreckten Bereich (14) mit proximalen (12) und distalen (20) Endbereichen und einer Längsachse;
ein Paar von Klemmbacken (124), die sich beide auf einer längs verlaufenden Klemmbackenachse von dem distalen Endbereich erstrecken; und
eine Mehrzahl von Befestigern (210), die innerhalb jeder der Klemmbacken angeordnet sind, wobei das Instrument dadurch gekennzeichnet ist, daß:
i. das Instrument zur endoskopischen Chirurgie geeignet ist;
ii. der langgestreckte Bereich (14) an einem Schwenkgelenk (16) so gelenkig ist, daß das distale Ende (20) des langgestreckten Bereiches und das Paar von Klemmbacken (124) sich am Gelenk (16) von der Längsachse weg abbiegen können.

2. Instrument gemäß Anspruch 1, weiter umfassend zumindest ein Nahtmaterial (300), das zumindest teilweise neben dem Paar von Klemmbacken angeordnet ist.

3. Instrument gemäß Anspruch 1 oder 2, weiter umfassend eine Einrichtung (16), um das Paar von Klemmbacken relativ zu Längsachse des endoskopischen Bereiches zu verschwenken, wobei die längsverlaufende Klemmbackenachse jeder der Klemmbacken im wesentlichen parallel zur endoskopischen Längsachse ist, wenn das Instrument in einer nicht abgelenkten Position ist.

4. Instrument gemäß Anspruch 3, wobei, wenn das Instrument abgewinkelt ist, die längsverlaufende Klemmbackenachse in einem Winkel in bezug auf die Längsachse des endoskopischen Bereiches angeordnet ist.

5. Instrument gemäß einem der vorhergehenden Ansprüche, weiter umfassend einen Stent (450), der sich von dem langgestreckten Bereich am distalen Ende (20) erstreckt, wobei der Stent ein erstes Element (452) und ein zweites Element (454) umfaßt, die beide proximale und distale Enden besitzen, wobei die ersten und zweiten Elemente in Längsrichtung relativ zueinander so verschiebbar sind, daß der Längsabstand zwischen dem distalen Ende des ersten Elementes und dem proximalen Ende des zweiten Elementes einstellbar ist.

6. Instrument gemäß einem der vorhergehenen Ansprüche, weiter umfassend einen Stent (600), der sich von dem distalen Ende des langgestreckten Bereiches erstreckt, wobei der Stent umfaßt eine Mehrzahl (601 bis 604) von miteinander in Wechselwirkung stehenden Stentbereichen, die alle einen im allgemeinen ebenen Bereich (606) einstückig mit einem im allgemeinen gekrümmten Bereich (608) aufweisen, wobei die Stentbereiche so angeordnet sind, daß die im allgemeinen gekrümmten Bereiche entlang einer Längsachse ausgerichtet sind und zumindest einer der im allgemeinen ebenen Bereiche auf einer Seite der Längsachse und zumindest einer der im allgemeinen ebenen Bereiche auf der anderen Seite der Längsachse angeordnet sind.

7. Instrument gemäß Anspruch 6, wobei jeder der im allgemeinen gekrümmten Bereiche eine Längsöffnung (612) besitzt, die dazu angepaßt ist, um eine langgestreckte Stentstange durch diese hindurch aufzunehmen.

8. Instrument gemäß Anspruch 5, 6 oder 7, wobei der Stent zumindest teilweise aus bioabsorbierbarem Stoff hergestellt ist.

9. Instrument gemäß einem der vorhergehenden Ansprüche, weiter umfassend Einrichtungen zum Verformen der Befestiger.

10. Instrument gemäß einem der vorhergehenden Ansprüche, wobei zumindest eine der Klemmbacken ein feststehendes Plattenelement (732) und ein bewegbares Plattenelement (730) besitzt, wobei das bewegbare Plattenelement in Längsrichtung innerhalb der Klemmbacke verschiebbar ist, und beide Plattenelemente eine Mehrzahl von Schlitzen (730, 740) besitzen, die durch diese hindurchtreten;
ein Paar von Stangen (734) in der Längsrichtung innerhalb der Klemmbacke angeordnet sind, wobei die Stangen Vorsprünge aufweisen, die dazu angepaßt sind, zumindest teilweise durch die Mehrzahl von Plattenelementenschlitzen hindurchzutreten, wobei die Mehrzahl von Befestigern zwischen den Stangen angeordnet sind, und die Bewegung des bewegbaren Plattenelementes dazu führt, daß sich beide Stangen seitlich in Richtung aufeinander bewegen und dabei gleichzeitig die Befestiger verformen.

## Revendications

1. Instrument chirurgical (10) pour l'agrafage sans enclume d'une corde coulissante à du tissu, l'instrument comportant :
une partie oblongue (14) avec des portions d'extrémité proximale (12) et distale (20) et un axe longitudinal;
une paire de mâchoires (124), chacune s'étendant sur un axe de mâchoire longitudinale de ladite partie d'extrémité distale; et
plusieurs attaches (210) disposées dans chacune desdites mâchoires, l'instrument étant caractérisé en ce que :
i. l'instrument convient pour la chirurgie endoscopique;
ii. la partie oblongue (14) est articulée à un joint d'articulation (16) de telle sorte que ladite extrémité distale (20) de ladite partie oblongue et ladite paire de mâchoires (124) peuvent dévier à la jonction (16) au loin dudit axe longitudinal.

2. Instrument selon la revendication 1, comportant en outre au moins un fil de suture (300) au moins partiellement adjacent à ladite paire de mâchoires.

3. Instrument selon la revendication 1 ou 2, comportant en outre des moyens (16) pour articuler ladite paire de mâchoires relativement à l'axe longitudinal de ladite partie endoscopique, l'axe longitudinal des mâchoires de chacune desdites mâchoires étant sensiblement parallèle audit axe longitudinal endoscopique lorsque l'instrument se trouve dans une position non articulée.

4. Instrument selon la revendication 3, où lorsque ledit instrument est articulé, ledit axe de mâchoire longitudinale est positionné suivant un angle relativement à l'axe longitudinal de ladite partie endoscopique.

5. Instrument selon l'une des revendications précédentes, comportant en outre un extenseur (450) s'étendant depuis ladite extrémité distale (20) de la portion oblongue, ledit extenseur comportant un premier élément (452) et un deuxième élément (454) qui ont chacun des extrémités proximales et distales, où lesdits premiers et seconds éléments peuvent coulisser longitudinalement l'un par rapport à l'autre de telle sorte que la distance longitudinale entre l'extrémité distale dudit premier élément et l'extrémité proximale dudit deuxième élément est réglable.

6. Instrument selon l'une des revendications précédentes, comportant en outre un extenseur (600) s'étendant de l'extrémité distale de ladite partie oblongue, ledit extenseur comprenant plusieurs (601-4) parties d'extenseur en corrélation, chacune desdites parties ayant une partie généralement plane (606) intégrale avec une partie généralement courbée (608), lesdites parties d'extenseur étant agencées de façon que lesdites parties généralement courbées soient alignées le long d'un axe longitudinal et qu'au moins l'une desdites parties généralement planes soit disposée sur un côté dudit axe longitudinal et qu'au moins l'une desdites parties généralement planes soit disposée de l'autre côté dudit axe longitudinal.

7. Instrument selon la revendication 6, où chacune desdites parties généralement courbées a une ouverture longitudinale (612) apte à recevoir une tige oblongue de l'extenseur à travers celle-ci.

8. Instrument selon la revendication 5, 6 ou 7, où l'extenseur est au moins partiellement fabriqué en un matériau biorésorbable.

9. Instrument selon l'une des revendications précédentes, comprenant en outre des moyens pour déformer lesdites attaches.

10. Instrument selon l'une des revendications précédentes, où au moins l'une desdites mâchoires a un élément de plaque stationnaire (732) et un élément de plaque mobile (730), ledit élément de plaque mobile pouvant coulisser longitudinalement dans ladite mâchoire, chacun desdits éléments de plaque ayant plusieurs fentes (738, 740) passant à travers celui-ci;
une paire de barres (734) disposées longitudinalement dans ladite mâchoire, lesdites barres ayant des saillies aptes à passer au moins partiellement à travers ladite pluralité de fentes des éléments de plaque, où ladite pluralité d'attaches est disposée entre lesdites barres, et un déplacement dudit élément de plaque mobile amène chacune desdites barres à se déplacer latéralement les unes vers les autres en déformant ainsi simultanément lesdites attaches.
